# EUROPÄISCHE PATENTSCHRIFT

(11) **EP 1 438 929 B1**
(45) Veröffentlichungstag und Bekanntmachung des Hinweises auf die Patenterteilung: **15.08.2007**
(21) Anmeldenummer: 03025247.2
(22) Anmeldetag: 05.11.2003
(51) Int. Cl.: A61F 2/16

(54) **Vorrichtung zum Implantieren einer Intraokularlinse in ein Auge**
Device for implanting an intraocular lens in an eye
Dispositif pour implanter une lentille intraoculaire dans un oeil

(30) Priorität: 17.01.2003 DE 10301681
(43) Veröffentlichungstag der Anmeldung: 21.07.2004
(73) Patentinhaber: *Acri.Tec AG Gesellschaft für ophthalmologische Produkte, 16761 Hennigsdorf (DE)
(72) Erfinder: Serester, Alexander, Dipl.-Ing., 93077 Bad Abbach (DE); Fromberg, Ingeborg, 16567 Schönfliess (DE)
(74) Vertreter: Nöth, Heinz

(56) Entgegenhaltungen:
- WO-A-01/10352
- WO-A-02/26167
- WO-A-97/15253

## Beschreibung

Die Erfindung betrifft eine Vorrichtung zum Implantieren einer Intraokularlinse in ein Auge nach dem Oberbegriff des Patentanspruches 1.

Eine derartige aus WO 97/15253 A bekannte Vorrichtung enthält eine Injektionskartusche, in deren Kartuschenlumen die zu implantierende Linse im gefalteten oder gerollten Zustand angeordnet wird. Die Kartusche ist so ausgebildet, dass mit ihr die Linse auch gefaltet oder gerollt werden kann. Mittels eines durch das Kartuschenlumen schiebbaren Stößels wird die Linse beim Implantieren durch das in das Auge eingeführte distale Kartuschenende an den Implantationsort geschoben. Das distale Stößelende weist eine gummielastische Dichtung mit einer umlaufenden Dichtlippe auf, welche an der Innenwand des Katuschenlumens anliegt. Beim Verschieben der Linse durch das Kartuschenvolumen in Richtung zum distalen Kartuschenende hin wird dabei verhindert, dass Teile der Linse, insbesondere fadenförmige Linsenhaptikteile zwischen der Innenwand des Kartuschenlumens und dem distalen Stößelende eingeklemmt werden.

Aufgabe der Erfindung ist es, eine Vorrichtung der eingangs genannten Art zu schaffen, mit welcher ein unbehindertes Verschieben der Linse im Kartuschenlumen und ein Nachaußenschieben der zu implantierenden Linse durch das distale Kartuschendende mit verringerter Reibung zwischen der Mantelfläche der Dichtung und der Innenwand des Kartschenlumens erreicht wird. Diese Aufgabe wird erfindungsgemäß durch die Merkmale des Patentanspruches 1 gelöst.

Die Unteransprüche beinhalten vorteilhafte Weiterbildungen der Erfindung.

Bei der Erfindung weist die Dichtung am distalen Stößelende eine umlaufende Dichtlippe auf, die an der Innenwand des Kartuschenlumens anliegt. In vorteilhafter Weise umgibt die Dichtlippe, welche Ringform besitzt, eine konkave Ausnehmung am distalen Dichtungsende. Vorzugsweise besitzt die Dichtlippe an ihrem freien Ende einen konischen Querschnitt.

Beim Verschieben des distalen Stößelendes in Richtung zum distalen Kartuschenende hin wird die Dichtlippe in vorteilhafter Weise durch einen am distalen Dichtungsende entstehenden Druck, insbesondere Staudruck, radial nach außen gedrückt, so dass sie dicht an der Innenwand des Kartuschenlumens anliegt. Die Gefahr, dass dünne Linsenteile, insbesondere Haptikteile zwischen die Innenwand des Kartuschenlumens und das distale Stößelende eingeklemmt werden, ist hierdurch beseitigt. Der Staudruck am distalen Dichtungsende entsteht insbesondere **dadurch**, dass in das Kartuschenlumen für das erleichterte Verschieben der gefalteten oder gerollten Linse eine Flüssigkeit, beispielsweise viskoelastische Flüssigkeit oder isotonische Lösung oder dergleichen eingebracht ist, durch die der Querschnitt des Kartuschenlumens gegenüber dem distalen Kartuschenende dicht verschlossen ist. Beim Verschieben des Stößels mit der erfindungsgemäß ausgebildeten Dichtung am distalen Stößelende entsteht der beschriebene Staudruck, welcher zur selbstdichtenden Anlage der Dichtlippe und/oder des Dichtungsmaterials an der Innenwand des Kartuschenlumens führt. Durch den Staudruck wird im Dichtungsmaterial ein radial nach außen gerichteter Druck induziert bzw. erzeugt. Dieser radiale Druck wirkt im vorderen (distalen) Bereich der Dichtung.

Durch die konkave Ausnehmung wird dieser auf die Dichtlippe radial nach außen gerichteter Druck noch unterstützt. Die konkave Ausnehmung kann einen gekrümmten Teilkreis oder parabelförmigen Querschnitt, rechteckigen oder trapezförmigen Querschnitt oder auch einen etwa konisch zulaufenden Querschnitt oder ähnlich gestalteten Querschnitt aufweisen.

Die zylindrische Mantelfläche der Dichtung weist zwei unterschiedliche Durchmesserbereiche auf, welche durch eine umlaufende Stufe in der Mantelfläche gebildet sind. Der Mantelflächenbereich mit dem größeren Durchmesser schließt sich dabei an die Dichtlippe an. Der zylindrische Mantelflächenbereich mit dem kleineren Durchmesser folg der umlaufenden Stufe. Durch diese Abstufung in der Mantelfläche der Dichtung wird eine Reibungsentlastung erreicht. Die Stufenhöhe beträgt ca. 0,1 mm. Die Stufenhöhe kann hiervon abweichen und ist so bemessen, dass die gewünschte Reibungsentlastung erreicht wird.

Anhand der Figuren wird an Ausführungsbeispielen die Erfindung noch näher erläutert.

Es zeigt
- Fig. 1: eine schnittbildliche Darstellung einer Injektionskartusche mit distalem Stößelende;
- Fig. 2: ein erstes Ausführungsbeispiel einer Dichtung;
- Fig. 3: ein zweites Ausführungsbeispiel einer Dichtung; und
- Fig. 4: ein drittes Ausführungsbeispiel einer Dichtung;

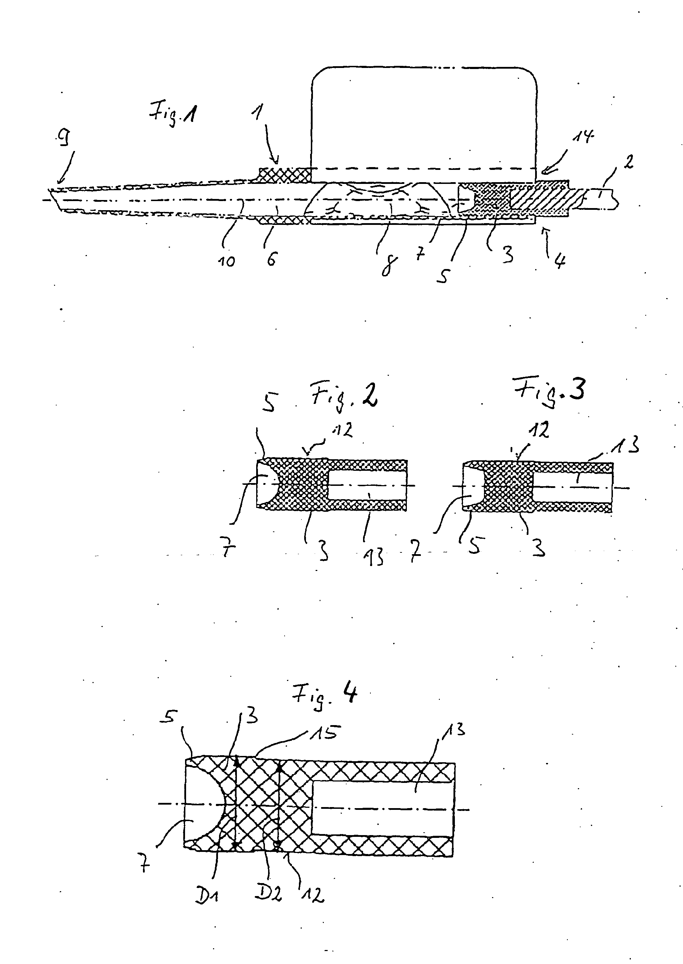

Eine schnittbildlich in der Fig. 1 dargestellte Injektionskartusche 1 enthält ein Kartuschenlumen 6, welches am distalen Kartuschenende 9 und am entgegengesetzt dazu liegenden proximalen Kartuschenende 14 offen ist. Durch das proximale offene Lumenende ist ein distales Stößelende 4 eines Stößels 2 in das Kartuschenlumen 6 eingeschoben. Am distalen Stößelende 4 befindet sich eine Dichtung 3. Beim dargestellten Ausführungsbeispiel besteht die Dichtung 3 aus einem gummielastischen Stöpsel. In den Fig. 2, 3 und 4 sind Ausführungsformen dieses die Dichtung 3 bildenden gummielastischen Stöpsels dargestellt. Das distale Stößelende 4 ragt in eine Verankerungsöffnung 13 des Stöpsels.

Die Injektionskartusche 1 ist vorzugsweise in der Weise ausgebildet, dass mit ihr, ähnlich wie es in der US 4,681,102 beschrieben ist, die zu implantierende Linse 8 gefaltet werden kann. Nach dem Falten bzw. Rollen der zu implantierenden Intraokularlinse 8 befindet sich diese im Kartuschenlumen 6, wie es schematisch in der Fig. 1 dargestellt ist. Zum erleichterten Verschieben der Linse 8 in Richtung zum distalen Kartuschenende 9 hin, wird in das Kartuschenlumen 6 eine viskoelastische Lösung eingebracht.

Durch Verschieben des Stößels 2 in Richtung zum distalen Kartuschenende 9 hin wird die Linse 9 durch das Kartuschenlumen 6 transportiert.

Um zu verhindern, dass dünne Linsenteile, insbesondere Haptikteile zwischen die Innenwand des Kartuschenlumens 6 und eine Mantelfläche 12 der Dichtung 3 eingeklemmt werden, ist die Dichtung 3 mit einer umlaufenden Dichtlippe 5 ausgestattet. Die Dichtlippe 5 wird vor allem durch den beim Verschieben des Stößels 2 sich am distalen Dichtungsende ausbildenden Staudruck radial nach außen gegen die Innenwand des Kartuschenlumens 6 gedrückt. Durch diesen Selbstdichtungseffekt wird verhindert, dass dünne Linsenteile, insbesondere die fädchenförmige Haptik zwischen die Innenwand des Kartuschenlumens und das distale Stößelende 4, insbesondere die Mantelfläche 12 der Dichtung 3 eingeklemmt werden. Auf diese Weise wird ein behinderungsfreier Transport der Linse 8 zum distalen Kartuschenende 9 hin und durch die dort befindliche Öffnung hindurch in den Implantationsort im Auge gewährleistet.

In den Fig. 2, 3 und 4 sind verschiedene Ausführungsformen für die als Stöpsel ausgebildete Dichtung 3 dargestellt.

Die Querschnitte der als Stöpsel ausgebildete Dichtungen 3 in den Fig. 2 bis 4 sind an den Querschnitt des Kartuschenlumens 6 angepasst. In bevorzugter Weise sind diese kreisrunde Querschnitte.

Bei den Ausführungsbeispielen der Fig. 2 und 3 sind an den distalen Enden der die Dichtung 3 bildenden Stöpsel konkave Ausnehmungen 7 vorgesehen. Bei dem in Fig. 2 dargestellten Ausführungsbeispiel ist die Ausnehmung 7 in Form einer Kugelhalbschale ausgebildet. In der Fig. 3 besitzt die konkave Ausnehmung 7 die Form eines Trapezoids. Die konkave Ausnehmung 7 kann auch eine andere Gestalt haben. Wesentlich ist, dass der Rand der Ausnehmung 7 von der Dichtlippe 5 umfasst wird. Vorzugsweise besitzt die Dichtlippe 5 einen konisch zulaufenden Querschnitt. Hierzu ist wenigstens die die konkave Ausnehmung 7 begrenzende Fläche schräg verlaufend ausgebildet, wie es in den Fig. 2 und 3 dargestellt ist. Ferner kann, wie es die Ausführungsbeispiele der Fig. 2 und 3 zeigen, auch die Außenfläche der Dichtlippe, welche in die Mantelfläche 12 übergeht, abgeschrägt sein.

Die Dichtung 3 besteht vorzugsweise aus einem gummielastischen Material. Dieses Material ist so beschaffen, dass beim Verschieben des Stößels 2 in der Fig. 1 von rechts nach links, d.h. zum distalen Kartuschenende 9 hin, die Dichtlippe 5 radial nach außen verformt wird, so dass eine dichte Anlage an der Innenwand des Kartuschenlumens 6 erreicht wird.

Wie schon erläutert, kann der Querschnitt des Kartuschenlumens 6 kreiszylindrisch ausgebildet sein und die Lumenachse 10, entlang welcher die Linse 9 durch die Stößelbewegung transportiert wird, aufweisen. Wie aus der Fig. 1 zu ersehen ist, verengt sich das Kartuschenlumen 6 zum distalen Kartuschenende 9 hin. Vorzugsweise ist in diesem Bereich die Innenwand des Kartuschenlumens 6 konisch ausgebildet. In diesem verengten Kartuschenlumen 6 wird der Querschnitt der gefalteten und/oder gerollten Linse 8 weiter verringert. Die Linse 8 wird dann durch die distale Öffnung der Kartusche zusammen mit der viskosen Flüssigkeit in den Implantationsort, beispielsweise eine Linsenkapsel des Auges eingebracht.

Beim Ausführungsbeispiel der Figur 4 sind in der kreiszylindrischen Mantelfläche zwei Mantelflächenbereich vorgesehen, die unterschiedliche Durchmesser D1 und D2 aufweisen. Zwischen beiden Mantelflächenbereichen befindet sich eine umlaufende Stufe 15. Der Mantelflächenbereich mit dem größeren Durchmesser D1 schließt sich an die Dichtlippe 5 an. Beim dargestellten Ausführungsbeispiel der Figur 4 ist am distalen Dichtungsende die konkave Ausnehmung 7 vorgesehen, wobei die Dichtlippe 5 konisch zuläuft. Die gestufte Mantelfläche kommt jedoch auch bei den anderen Ausführungsbeispielen der Figuren 2 und 3 zur Anwendung.

Der Durchmesser der Dichtung hängt von dem Innendurchmesser des Kartuschenlumens 6 ab. In der Regel ist dieser Durchmesser geringer als 3 mm, um die gefaltete bzw. gerollte Linse 8 durch einen möglichst kleinen Schnitt in das Auge zu implantieren.

Die Stufe 15 liegt vor dem distalen Ende der Verankerungsöffnung 13, wie aus der Figur 4 zu ersehen ist. Der Abstand vom distalen Ende der Dichtung 3 bis zur Abstufung kann etwa 2 mm betragen. Der Abstand des distalen Endes der Dichtung 3 bis zum distalen Ende der Verankerungsöffnung 13 kann etwa 3,5 mm betragen. Die erfindungsgemäße Dichtung ist jedoch nicht auf diese Abmessungen beschränkt.

### [Bezugszeichenliste]

- 1: Injektionskartusche
- 2: Stößel
- 3: Dichtung (Stöpsel)
- 4: distales Stößelende
- 5: Dichtlippe
- 6: Kartuschenlumen
- 7: konkave Ausnehmung
- 8: Intraokularlinse
- 9: distales Kartuschenende
- 10: Lumenachse
- 11: distale ebene Fläche der Dichtung
- 12: Mantelfläche der Dichtung
- 13: Verankerungsöffnung
- 14: proximales Ende der Kartusche
- 15: umlaufende Stufe

## Patentansprüche

1. Vorrichtung zum Implantieren einer Intraokularlinse (8) in ein Auge mit einer Injektionskartusche (1) und einem durch ein Kartuschenlumen (6) schiebbaren Stößel(2), mit welchem die Linse durch ein distales Kartuschenende (9) schiebbar ist und welcher an seinem distalen Stößelende (4) eine Dichtung (3) aufweist, die mit einer Mantelfläche (12) an der Innenwand des Kartschuschenlumens (9) anliegt, wobei die Dichtung (3) an ihrem distalen Ende eine umlaufende Dichtlippe (5) aufweist, die an der Innenwand des Kartschenlumens (6) anliegt,
**dadurch gekennzeichnet, dass** die Mantelfläche (12) eine umlaufende Stufe (15) aufweist, so dass zwei Mantelflächenbereiche mit unterschiedlichen Durchmessern (D1, D2) gebildet sind, und der Mantelflächenbereich mit dem größeren Durchmesser (D1) sich an die Dichtlippe (5) anschließt.

2. Vorrichtung nach Anspruch 1,
**dadurch gekennzeichnet, dass** die Dichtlippe (5) eine konkave Ausnehmung (7) am distalen Dichtungsende umgibt.

3. Vorrichtung nach Ansprüch 1 oder 2,
**dadurch gekennzeichnet, dass** die Dichtlippe (5) an ihrem freien Ende einen konischen Querschnitt aufweist.

4. Vorrichtung nach einem der Ansprüche 1 bis 3, geeignet ist,
**dadurch gekennzeichnet, dass** die Dichtlippe (5) durch einen beim Verschieben im Kartuschenhohlraum am distalen Dichtungsende (7; 11) entstehenden Druck radial nach außen gedrückt zu werden.

5. Vorrichtung nach einem der Ansprüche 1 bis 4,
**dadurch gekennzeichnet, dass** die Dichtung (3) als Stöpsel ausgebildet ist.

6. Vorrichtung nach einem der Ansprüche 1 bis 5,
**dadurch gekennzeichnet, dass** das Kartuschenlumen (6) am distalen und am proximalen Ende der Kartusche offen ist.

7. Vorrichtung nach einem der Ansprüche 1 bis 6,
**dadurch gekennzeichnet, dass** das Kartuschenlumen (6) zum distalen Kartuschenende (9) hin sich konisch verjüngt.

## Claims

1. A device for implanting an intraocular lens (8) in an eye comprising an injection cartridge (1) and a plunger (2) which is displaceable through a cartridge lumen (6) and with which the lens is displaceable through a distal cartridge end (9) and which at its distal plunger end (4) has a seal (3) which bears with a peripheral surface (12) against the inside wall of the cartridge lumen (6), wherein the seal at its distal end has a peripherally extending sealing lip (5) which bears against the inside wall of the cartridge lumen (6),
**characterised in that** the peripheral surface (12) has a peripherally extending step (15) so that two peripheral surface regions of different diameters (D1, D2) are formed and the peripheral surface region of the larger diameter (D1) adjoins the sealing lip (5).

2. A device according to claim 1 **characterised in that** the sealing lip (5) surrounds a concave recess (7) at the distal end of the seal.

3. A device according to claim 1 or claim 2 **characterised in that** the sealing lip (5) is of a conical cross-section at its free end.

4. A device according to one of claims 1 to 3 **characterised in that** the sealing lip (5) is suitable for being pressed radially outwardly by a pressure which is produced upon displacement in the cartridge interior at the distal end of the seal (7; 11).

5. A device according to one of claims 1 to 4 **characterised in that** the seal (3) is in the form of a stopper.

6. A device according to one of claims 1 to 5 **characterised in that** the cartridge lumen (6) is open at the distal end and the proximal end of the cartridge.

7. A device according to one of claims 1 to 6 **characterised in that** the cartridge lumen (6) tapers conically towards the distal cartridge end (9).

## Revendications

1. Dispositif pour implanter une lentille intraoculaire (8) dans un oeil, comportant une cartouche d'injection (1) et un coulisseau (2) pouvant être inséré à travers une lumière de cartouche, avec lequel la lentille peut être insérée par une extrémité de cartouche distale (9) et qui présente à son extrémité de coulisseau distale (4) un joint d'étanchéité (3) qui repose par une surface d'enveloppe (12) sur la paroi intérieure de la lumière de cartouche (9), le joint d'étanchéité (3) présentant à son extrémité distale une lèvre d'étanchéité périphérique (5) qui repose sur la paroi intérieure de la lumière de cartouche (6),
**caractérisé en ce que** la surface d'enveloppe (12) présente un décrochement périphérique (15), de sorte que deux zones de surface d'enveloppe de diamètres différents (D1, D2) sont formées, et la zone de surface d'enveloppe présentant le plus grand diamètre (D1) fait suite à la lèvre d'étanchéité (5).

2. Dispositif selon la revendication 1,
**caractérisé en ce que** la lèvre d'étanchéité (5) entoure un évidement concave (7) à l'extrémité de joint distale.

3. Dispositif selon la revendication 1 ou 2,
**caractérisé en ce que** la lèvre d'étanchéité (5) présente à son extrémité libre une section transversale conique.

4. Dispositif selon l'une des revendications 1 à 3,
**caractérisé en ce que** la lèvre d'étanchéité (5) est apte à être pressée radialement vers l'extérieur par une pression générée à l'extrémité de joint distale (7 ; 11) lors du déplacement à l'intérieur de l'espace creux de la cartouche.

5. Dispositif selon l'une des revendications 1 à 4,
**caractérisé en ce que** le joint d'étanchéité (3) est réalisé sous la forme d'un bouchon.

6. Dispositif selon l'une des revendications 1 à 5,
**caractérisé en ce que** la lumière de cartouche (6) est ouverte à l'extrémité distale et à l'extrémité proximale de la cartouche.

7. Dispositif selon l'une des revendications 1 à 6,
**caractérisé en ce que** la lumière de cartouche (6) rétrécit de manière conique en direction de l'extrémité de cartouche distale (9).
